# DEMANDE DE BREVET EUROPEEN

(11) **EP 2 277 460 A1**
(43) Date de publication de la demande: **26.01.2011**
(21) Numéro de dépôt: 10170327.0
(22) Date de dépôt: 21.07.2010
(51) Int. Cl.: A61B 17/15, A61B 17/17, A61F 2/46, A61B 17/02, A61B 19/00, A61F 2/00, A61F 2/02, A61F 2/30

(54) **Instrumentation chirurgicale pour la préparation à la pose d'une prothèse de genou**

(30) Priorité: 24.07.2009 FR 0955205
(71) Demandeur: TORNIER, 38330 Saint-Ismier (FR)
(72) Inventeur: Faure, M. Eric, 38190, LAVAL (FR); Rochetin, Olivier, 42130, MARCILLY-LE-CHATEL (FR)
(74) Mandataire: Grand, Guillaume

(57) **Abrégé**

Cette instrumentation (1) comporte :
- une pièce d'appui tibial (10) et une pièce d'appui fémoral (20), adaptées pour s'appuyer respectivement contre l'extrémité supérieure du tibia (T) et contre les condyles postérieurs du fémur (F), de manière à écarter l'un de l'autre le tibia et le fémur lorsque le genou est fléchi, et
- des moyens (30) de montage et de couplage de la pièce d'appui fémoral sur la pièce d'appui tibial, conçus pour ajuster la rotation externe du fémur sélectivement en fonction d'un critère relatif à une technique de pose, parmi au moins deux critères appartenant à un groupe qui inclut la mesure peropératoire de la valeur de la rotation externe, la fixation préopératoire à une valeur de rotation prédéterminée, la position de la ligne de Whiteside, la position de la ligne biépycondylienne et la position du condyle interne.

## Description

La présente invention concerne une instrumentation chirurgicale pour la préparation à la pose d'une prothèse de genou.

L'invention s'intéresse en particulier aux difficultés de positionnement antéropostérieur du composant fémoral d'une prothèse de genou, eu égard à ce qui est couramment appelé la rotation externe du fémur par rapport au tibia du patient opéré, c'est-à-dire à la position angulaire du fémur autour de son axe longitudinal. En effet, la pose d'un composant prothétique fémoral nécessite de réaliser, entre autres, des coupes antérieure et postérieure du fémur, les plans de coupe obtenus étant dimensionnés pour former des appuis plans correspondants pour la fixation du composant fémoral. II en résulte que, si les plans de coupe sont réalisés sans tenir compte de la rotation externe du fémur, en particulier sans prendre la précaution soit d'orienter les plans de coupe avec la même valeur de rotation externe que le fémur, soit de réaliser des corrections chirurgicales osseuses et/ou ligamentaires pour ajuster la valeur de rotation externe du fémur, le composant fémoral implanté risque de conduire à une subluxation ou luxation de la rotule du patient.

En pratique, plusieurs techniques chirurgicales sont actuellement connues pour évaluer et tenir compte de la rotation externe du fémur lors de la pose d'une prothèse de genou. Selon les usages et les « écoles » des chirurgiens, ainsi que selon les cas cliniques traités, l'une de ces différentes techniques est préconisée, avec, pour leur mise en oeuvre, autant d'instrumentations correspondantes. Autrement dit, à chaque technique de pose, est associée une instrumentation spécifique, comme cela est proposé respectivement par US-A-2007/293868, par US-A-5 860 980, par US-A-2004/122441, par US-A-2007/162036 et par US-A-5 830 216.

Le but de la présente invention est de proposer une instrumentation qui, pour les chirurgiens, facilite la prise en compte de la rotation externe du fémur lors de la préparation à la pose d'une prothèse de genou.

A cet effet, l'invention a pour objet une instrumentation chirurgicale pour la préparation à la pose d'une prothèse de genou, telle que définie à la revendication 1.

L'idée à la base de l'invention est de mettre à disposition des chirurgiens une seule et même instrumentation grâce à laquelle la rotation externe du fémur pourra être ajustée selon une technique parmi au moins deux, voire trois, voire quatre, voire les cinq techniques possibles parmi les techniques correspondant aux cinq critères du groupe défini à la revendication 1. Grâce à cette seule et même instrumentation chirurgicale, chaque chirurgien choisit, s'il le souhaite au cours même de l'intervention chirurgicale proprement dite, le critère qu'il souhaite pour ajuster la rotation externe du fémur. Cet ajustement est réalisé avec efficacité et précision grâce au fait qu'il est mis en oeuvre alors que l'articulation du genou subit une distraction sous l'action de la pièce d'appui tibial et de la pièce d'appui fémoral. En pratique, à cet effet, la pièce d'appui tibial est mise en action après résection de l'extrémité supérieure du tibia tandis que la pièce d'appui fémoral est mise en action après résection distale du fémur. Ainsi, après avoir mis en tension relative le tibia et le fémur, le genou étant préalablement fléchi à 90° pour permettre la mise en place des pièces d'appui, le chirurgien peut apprécier visuellement la rotation externe du fémur et la mesurer et/ou décider, le cas échéant, de mettre en oeuvre des corrections chirurgicales osseuses et/ou ligamentaires pour ajuster cette rotation externe en fonction d'un critère qu'il choisit, à sa discrétion, parmi le groupe de critères disponibles. Bien entendu, s'il le souhaite, le chirurgien peut ajuster la rotation externe du fémur de manière successive suivant deux critères parmi ceux du groupe, à titre de vérification ou de comparaison.

Des caractéristiques avantageuses de l'instrumentation conforme à l'invention, prises isolément ou selon toutes les combinaisons techniquement possibles, sont spécifiées aux revendications 2 à 15.

L'invention sera mieux comprise à la lecture de la description qui va suivre, donnée uniquement à titre d'exemple et faite en se référant aux dessins sur lesquels :
- les figures 1A, 1B et 1C illustrent un premier temps opératoire, la figure 1A étant une vue en perspective d'une instrumentation chirurgicale conforme à l'invention, en cours d'utilisation, tandis que les figures 1B et 1C sont des vues en élévation selon respectivement les flèches IB et IC de la figure 1A ;
- les figures 1D et 1E sont des coupes de l'instrumentation des figures 1A à 1C, montrée seule, selon respectivement les lignes ID-ID et IE-IE de la figure 1B ;
- la figure 2 est une vue en perspective de l'instrumentation, illustrant un deuxième temps opératoire ;
- les figures 3A, 3B, 3C et 3D sont des vues en perspective de l'instrumentation chirurgicale, illustrant respectivement quatre possibilités de mise en oeuvre d'un troisième temps opératoire ;
- les figures 4A et 4B illustrent un quatrième temps opératoire, la figure 4A étant une vue en perspective de l'instrumentation chirurgicale tandis que la figure 4B est une vue en élévation d'une partie seulement de cette instrumentation ;
- la figure 5 est une vue en perspective de l'instrumentation, illustrant un cinquième temps opératoire ;
- la figure 6 illustre un sixième temps opératoire, en montrant un bloc de coupe fémorale positionné grâce à l'instrumentation des figures précédentes ; et
- la figure 7 est une vue analogue à la figure 5, illustrant une variante de mise en oeuvre du cinquième temps opératoire grâce à l'instrumentation conforme à l'invention.

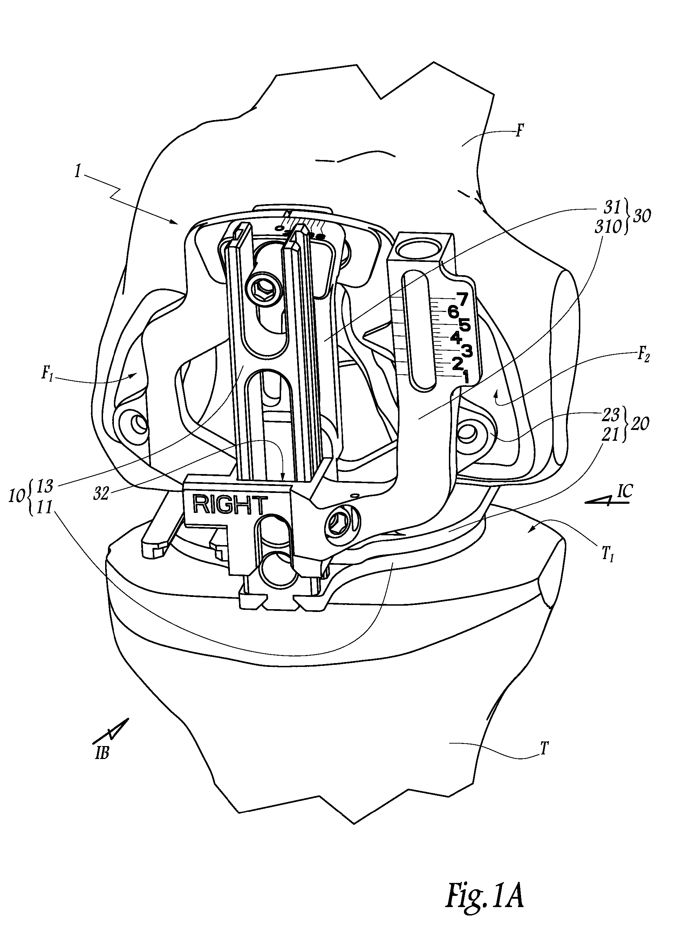

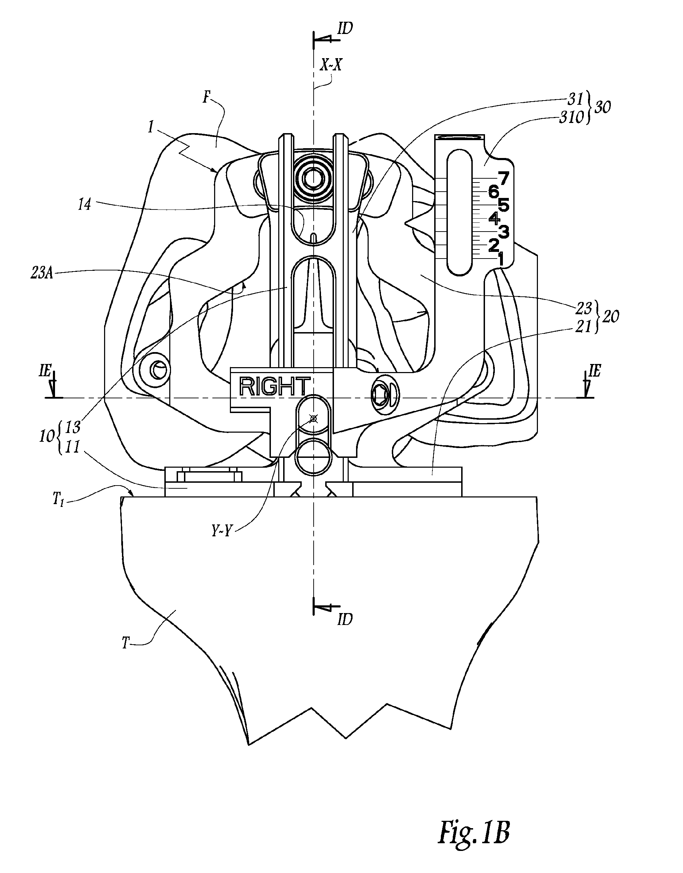

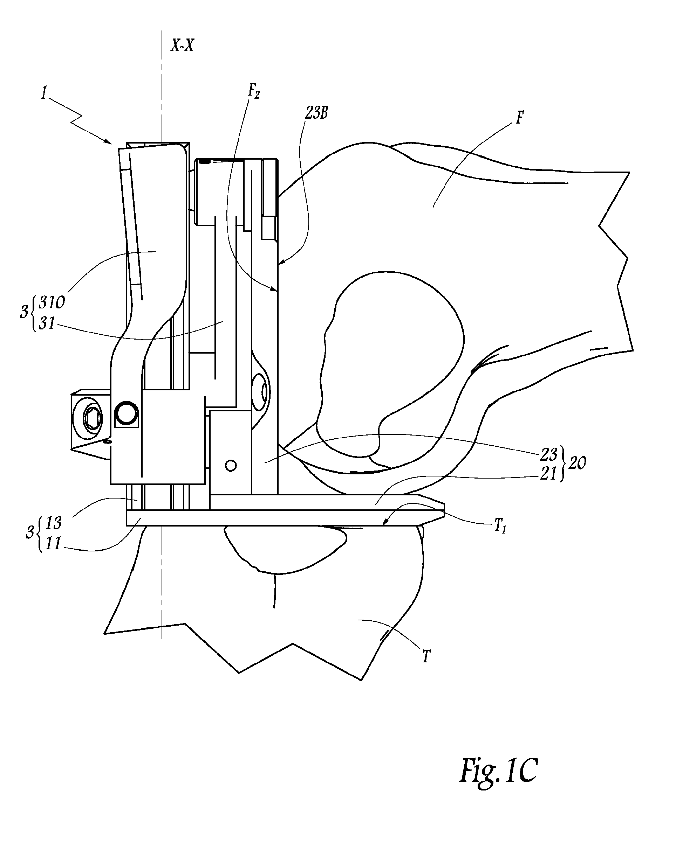

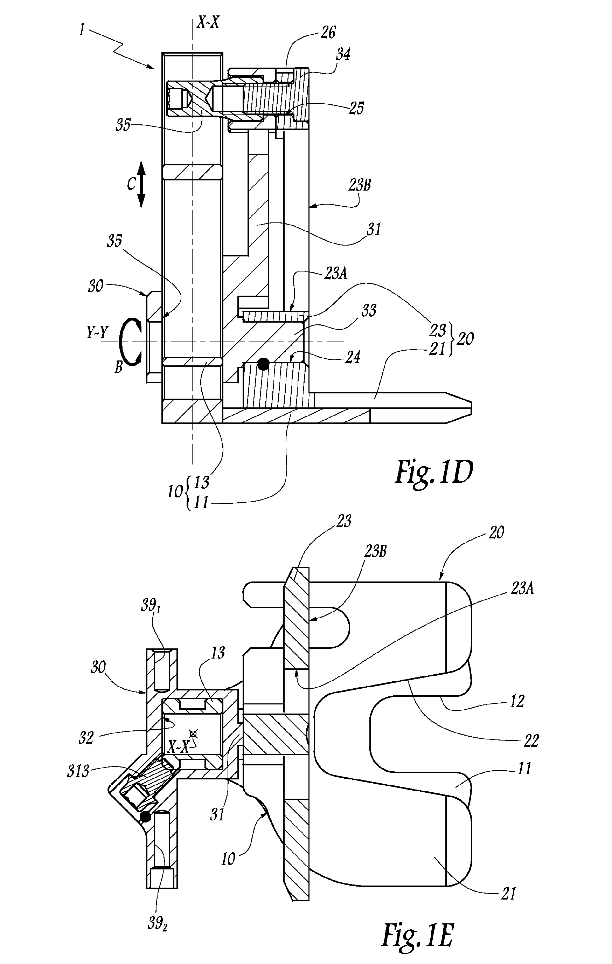

Sur les figures 1 à 7 est représentée une instrumentation chirurgicale 1 comprenant plusieurs composants qui vont être détaillés les uns après les autres, au fur et à mesure de la description d'une utilisation de cette instrumentation pour la préparation à la pose d'une prothèse de genou, non représentée, entre un tibia T et un fémur F d'un patient. Cette utilisation de l'instrumentation 1 va être présentée en six temps opératoires, réalisés de manière successive.

Par ailleurs, dans toute la suite, les termes « supérieur », « postérieur », « distal », etc. s'entendent dans leur sens anatomique, en considérant que le patient opéré se tient debout sur une surface horizontale.

Préalablement au premier temps opératoire montré sur les figures 1A à 1C, un chirurgien incise les chairs molles entourant l'articulation du genou entre le tibia T et le fémur F, afin d'accéder à l'extrémité supérieure du tibia et à l'extrémité inférieure du fémur. De manière connue en soi, le chirurgien résèque alors, d'une part, l'extrémité supérieure du tibia, selon un plan de résection sensiblement horizontal, de manière à former ainsi une surface plane T₁ à l'extrémité supérieure du tibia T, et, d'autre part, l'extrémité inférieure du fémur F, selon un plan de résection également horizontal alors que le genou du patient est en extension, de manière à former deux surfaces planes distales F₁ et F₂ à l'extrémité inférieure du fémur F, respectivement délimitées par les condyles distaux externe et interne.

Pour passer au premier temps opératoire, le chirurgien fléchit ensuite le genou du patient opéré, en positionnant le tibia T et le fémur F à sensiblement 90° l'un de l'autre, comme montré sur les figures 1A à 1C. Le chirurgien met alors en place, entre l'extrémité supérieure du tibia T et l'extrémité inférieure du fémur F, un ensemble pré-assemblé de trois composants appartenant à l'instrumentation chirurgicale 1, à savoir une pièce d'appui tibial 10, une pièce d'appui fémoral 20 et une pièce 30 interposée mécaniquement entre les pièces 10 et 20. Ces trois pièces 10, 20 et 30 sont représentées seules sur les figures 1D et 1E.

Ainsi, la pièce d'appui tibial 10 comprend une palette rigide 11 délimitant, sur sa face destinée à être tournée vers le bas en service, une surface plane 11A adaptée pour s'appuyer contre la surface tibiale T₁, en formant un contact plan-plan, comme bien visible sur les figures 1B et 1C. Comme bien visible sur la figure 1E, la palette 11 présente, dans sa partie postérieure, une échancrure 12 dimensionnée pour respecter, sans les endommager, les ligaments postérieurs du genou s'ils sont conservés. A son extrémité antérieure, la palette 11 est munie rigidement d'un pilier 13 qui s'étend en saillie, ici à la perpendiculaire, de la face de la palette opposé à la surface plane 11A. Ce pilier est centré sur un axe longitudinal, référencé X-X.

La pièce d'appui fémoral 20 comprend elle aussi une palette 21 qui délimite, sur sa face destinée à être tournée vers le haut en service, deux surfaces planes 21A, séparées l'une de l'autre par une échancrure postérieure 22 de la palette 21. Ces deux surfaces planes 21A sont adaptées pour s'appuyer contre les condyles postérieurs du fémur F, respectivement externe et interne, tandis que l'échancrure 22 respecte, sans les endommager, les ligaments postérieurs du genou s'ils sont conservés. A son extrémité antérieure, la palette 21 est munie rigidement d'une plaque 23 qui s'étend de manière saillante, ici à la perpendiculaire, de la face de la palette délimitant les surfaces 21A. Comme bien visible sur les figures 1B et 1D, cette plaque 23 est, dans sa région centrale, pourvue d'une ouverture traversante 23A permettant des observations chirurgicales à travers la plaque 23. Sur sa face postérieure, la plaque 23 délimite une surface plane 23B adaptée pour s'appuyer, en formant un contact plan-plan, contre les deux surfaces distales F₁ et F₂ du fémur F, comme montré sur la figure 1C.

La pièce 30 interposée entre les pièces d'appui tibial 10 et d'appui fémoral 20 comprend, quand à elle, un corps allongé 31 muni, à l'une de ses extrémités longitudinales destinée à être tournée vers le bas en service, d'un trou traversant 32 adapté pour être enfilé, de manière ajustée, autour du pilier 13 de la pièce 10. Par complémentarité de formes, le pilier 13 et le trou 32 forme une liaison coulissante selon l'axe X-X, comme indiqué par la double flèche C sur la figure 1D. De plus, à la même extrémité du corps 31 où est prévu le trou 32, ce corps est muni d'un pion saillant 33, qui s'étend depuis le côté postérieur du corps 31 suivant une direction perpendiculaire à l'axe X-X. Ce pion 33, qui est extérieurement cylindrique à base circulaire, en étant centré sur un axe Y-Y perpendiculaire à l'axe X-X, est reçu de manière ajustée dans un orifice complémentaire 24 délimité par la partie d'extrémité inférieure de la plaque 23, comme bien visible sur la figure 1D. La coopération entre l'orifice 24 et le pion 33 forme une liaison basculante autour de l'axe Y-Y, entre les pièces 20 et 30, comme indiqué par la double flèche courbe B sur la figure 1D. Dans le même temps, cet agencement lie les pièces 20 et 30 en translation selon l'axe X-X par rapport à la pièce 10. L'amplitude du montage basculant entre les pièces 20 et 30 est limitée par une fente traversante 25, délimitée à l'extrémité supérieure de la plaque 23 avec une direction longitudinale incurvée de manière centrée sur l'axe Y-Y. Cette fente 25 est traversée de part en part par un pion 34 rapporté fixement à l'extrémité du corps 31, opposée à celle où sont prévus le trou 32 et le pion 33. Avantageusement, pour des raisons qui apparaîtront plus loin, le pion précité 34 est prolongé de manière rectiligne par un téton 35 qui s'étend de manière saillante depuis la face antérieure du corps 31, comme montré sur la figure 1D.

Ainsi, lors du premier temps opératoire, le chirurgien manipule les pièces 10, 20 et 30 assemblées les unes aux autres dans une configuration dans laquelle la palette 21 est plaquée contre la palette 11, en coulissant au maximum vers le bas la pièce 30 par rapport à la pièce 10. Les palettes 11 et 21 sont conçues pour être plaquées l'une contre l'autre, en formant un contact plan-plan, de telle sorte que la pièce 20 occupe alors une position qualifiée de neutre en ce qui concerne son basculement autour de l'axe Y-Y. En pratique, la position basculée de la pièce 20 par rapport à la pièce 30 est repérée par une référence 26, matérialisée ici sous la forme d'une rainure délimitée dans la tranche d'extrémité supérieure de la pièce 20. Ainsi, dans la position de basculement neutre, la référence 26 s'étend dans un plan à la fois perpendiculaire aux palettes 11 et 21 et contenant l'axe X-X.

Après avoir luxé la rotule du patient, le chirurgien introduit les palettes 11 et 21 entre l'extrémité supérieure du tibia T et l'extrémité inférieur du fémur F, jusqu'à, d'une part, plaquer la surface 11A contre la surface plane tibiale T₁ et, d'autre part, plaquer la surface 23B contre les surfaces distales fémorales F₁ et F₂, comme montré sur les figures 1A, 1B et 1C. Les axes X-X et Y-Y se trouvent alors positionnés de manière respectivement perpendiculaire et parallèle à la direction longitudinale du fémur.

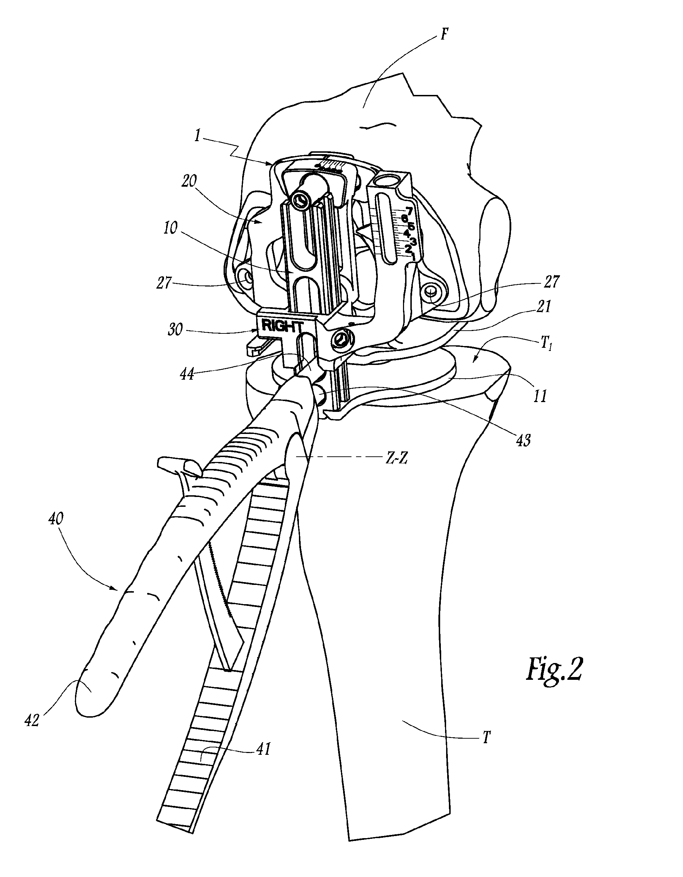

Dans un deuxième temps opératoire, montré à la figure 2, le chirurgien va distraire ou mettre en tension l'articulation du genou opéré, en écartant l'une de l'autre les pièces d'appui tibial 10 et d'appui fémoral 20. Pour ce faire, dans l'exemple de réalisation considéré ici, le chirurgien utilise une pince de distraction 40 qui comprend deux branches 41 et 42 articulées l'une par rapport à l'autre autour d'un axe Z-Z qui, en service, s'étend de manière sensiblement perpendiculaire à la fois à l'axe X-X et à l'axe Y-Y. Après avoir lié en mouvement, au moins suivant l'axe X-X, l'extrémité distale 43 de la branche 41 à la pièce d'appui tibial 10 et après avoir lié en mouvement, au moins suivant l'axe X-X, l'extrémité distale 44 de l'autre branche 42 à la pièce d'appui fémoral 20 via la pièce interposée 30, le chirurgien déplace l'une par rapport à l'autre les parties proximales respectives des branches 41 et 42 de manière à écarter l'une de l'autre les extrémités distales 43 et 44. Il en résulte que, par rapport à la pièce 10, la pièce 30 est entraînée en coulissement le long du pilier 13 suivant l'axe X-X et entraîne la pièce 20 dans un coulissement identique. La trajectoire de ce coulissement est imposée par la coopération entre le trou 32 et le pilier 13, et avantageusement, par la coopération entre le téton 35 et une gorge complémentaire 14, délimitée par l'extrémité libre du pilier 13 et ouverte vers le haut. Les palettes 11 et 21 s'éloignent alors l'une de l'autre selon une direction d'écartement sensiblement parallèle à l'axe X-X, comme montré sur la figure 2 : la palette 11 s'appuie vers le bas contre la surface plane tibiale T₁ tandis que la palette 21 s'appuie vers le haut contre les condyles postérieurs du fémur F. Ainsi, l'extrémité supérieure du tibia T et l'extrémité inférieure du fémur F s'éloignent l'une de l'autre, suivant une direction anatomique verticale, ce qui met en tension l'environnement ligamento-musculaire du genou opéré.

Avantageusement, avant de distraire le tibia T et le fémur F, la pièce d'appui fémoral 20 peut être solidarisée fixement au fémur, afin d'en stabiliser l'action sur cet os. Pour ce faire, dans l'exemple de réalisation considéré ici, la plaque 23 est munie, dans chacune de ses parties d'extrémité médiale et latérale, d'un orifice traversant 27 adapté pour recevoir chacun un élément d'ancrage osseux, non représenté, tel qu'une vis, une tige, une broche, etc.

Le réglage de l'intensité de la mise en tension entre le tibia T et le fémur F est soit laissé à la discrétion du chirurgien, soit contrôlé à l'aide de moyen de mesure millimétrique ou dynamométrique. Dans tous les cas, lorsque cette tension d'écartement atteint une valeur anatomiquement appropriée, les branches 41 et 42 sont bloquées l'une par rapport à l'autre, ce qui fige l'écartement relatif entre les pièces d'appui tibial 10 et d'appui fémoral 20.

Ainsi, à l'issue du deuxième temps opératoire, le fémur F est lié en mouvement à la pièce d'appui fémoral 20, en raison, d'une part, de l'appui pressant de la palette 21 contre les condyles postérieurs du fémur, sous l'action de la pince de distraction 40, et, d'autre part, par l'ancrage osseux d'éléments rapportés en travers des trous 27.

Dans un troisième temps opératoire, le chirurgien va ajuster la rotation externe du fémur F par rapport au tibia T. Cette rotation externe du fémur correspond à un mouvement rotatif du fémur sur lui-même, autour de l'axe longitudinal central du fémur, sous l'action de l'environnement osseux, ligamentaire et musculaire du fémur. Ainsi, lorsque le tibia T et le fémur F sont mis en tension relative à l'issue du deuxième temps opératoire, le fémur est susceptible de réaliser spontanément une rotation, en pratique de quelques degrés seulement, que le chirurgien va pouvoir ajuster en fonction d'un critère parmi plusieurs disponibles, grâce à l'instrumentation 1.

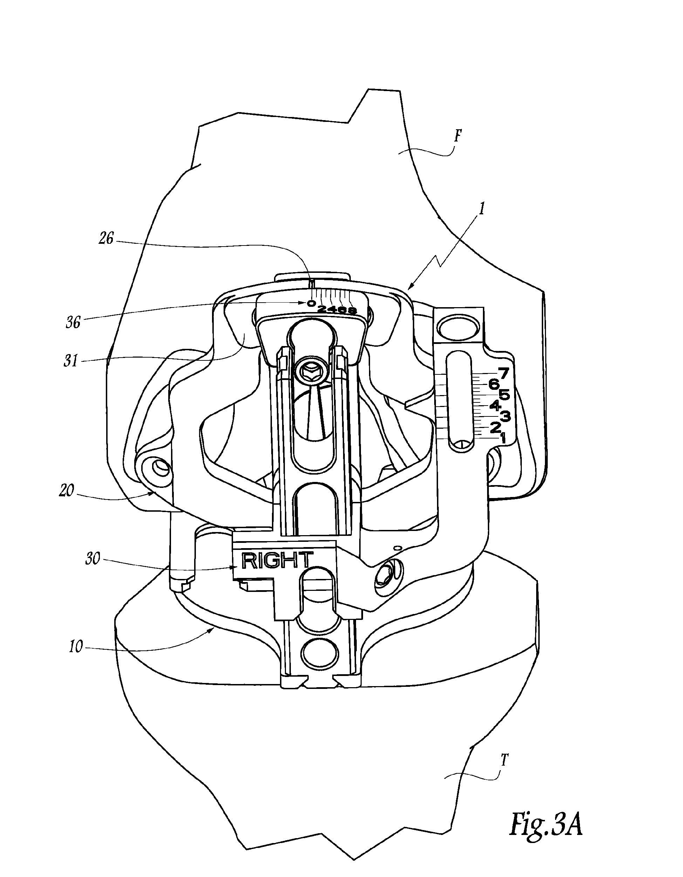

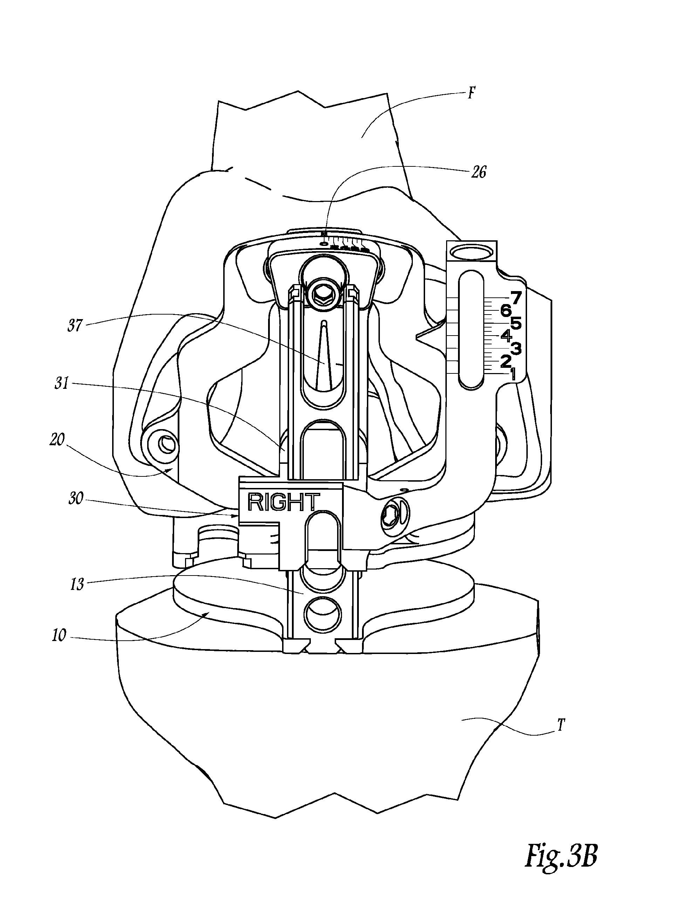

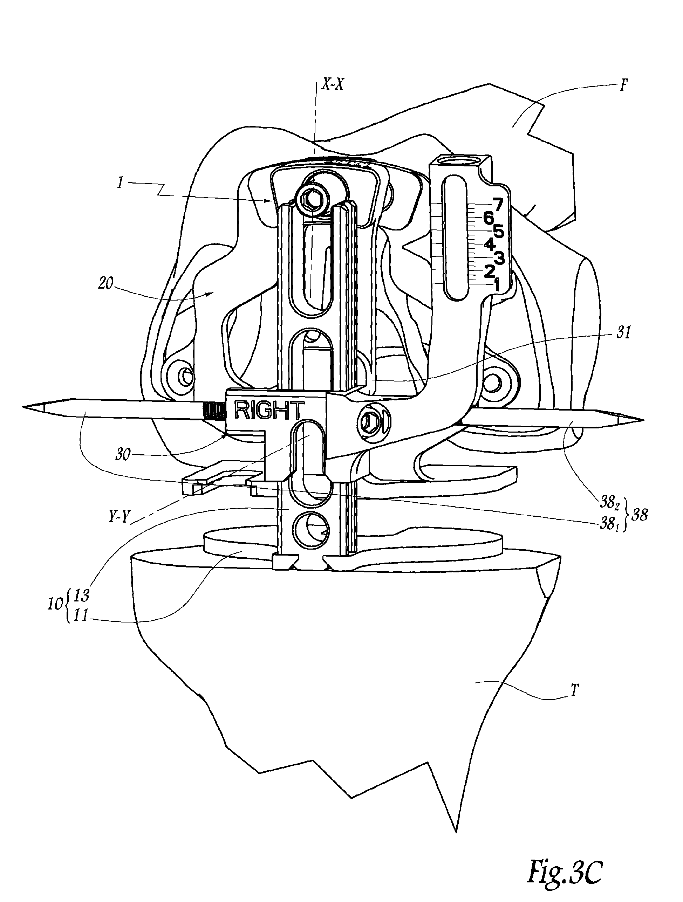

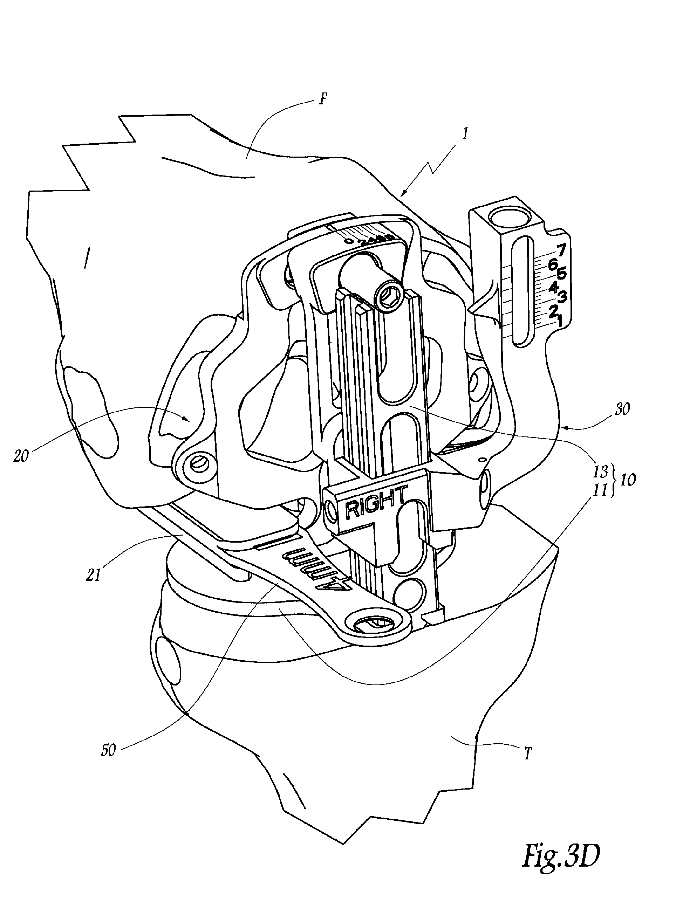

Pour illustrer cet aspect de l'instrumentation 1, le troisième temps opératoire évoqué ci-dessus est illustré par les figures 3A, 3B, 3C et 3D qui illustrent différentes possibilités d'ajustement de cette rotation externe du fémur, respectivement en fonction de critères distincts.

Ainsi, sur la figure 3A, le chirurgien a la possibilité de mesurer la valeur de la rotation externe du fémur F en fonction de la tension ligamento-musculaire du genou. A cet effet, la face d'extrémité supérieure du corps 31 de la pièce interposée 30 est munie de graduations 36, ici de degré en degré entre « 0 » et « 8 ». Dans l'exemple de réalisation considéré sur la figure 3A, ces graduations 36 sont portées à demeure par la pièce 30 mais, en variante non représentée, elles pourraient être rapportées de manière amovible. Dans tous les cas, ces graduations 36 sont réparties autour de l'axe de basculement Y-Y et sont conçues pour correspondre visuellement avec la référence 26 : en lisant la graduation parmi les graduations 36 située en regard de la référence 26, le chirurgien connaît en peropératoire la valeur angulaire du basculement de la pièce d'appui fémoral 20 par rapport à la pièce interposée 30 autour de l'axe Y-Y, ce qui lui fournit une mesure de la valeur de la rotation externe du fémur F puisque ce dernier est liée en mouvement à la pièce 20.

Si le chirurgien estime que la valeur de la rotation externe du fémur est trop importante, c'est-à-dire trop éloignée de la valeur de basculement neutre qui, en pratique, correspond à l'alignement de la référence 26 avec la graduation « 0 » des graduations 36, le chirurgien peut procéder à des corrections osseuses et/ou ligamentaires pour modifier cette valeur de rotation, jusqu'à obtenir une mesure peropératoire conforme à ses attentes.

Une variante, non représentée, de l'utilisation de l'instrumentation 1 pour ajuster la rotation externe du fémur F en fonction d'un autre critère que la mesure peropératoire de cette valeur de rotation externe, consiste à imposer, de manière préopératoire, une valeur prédéterminée pour la rotation externe du fémur. Dans ce cas, en pratique, le chirurgien détermine, avant l'intervention chirurgicale proprement dite, cette valeur prédéterminée sur la base de données préopératoires, fournies par exemple par des images de radiologie et/ou de scanner. Puis, au début du troisième temps opératoire, le chirurgien impose un basculement de la pièce d'appui fémoral 20 par rapport à la pièce interposée 30 autour de l'axe Y-Y, avec une valeur angulaire de basculement correspondant à la valeur de rotation externe prédéterminée. Pour ce faire, le chirurgien manipule le fémur F ou le tibia T, jusqu'à ce que la pièce 20 atteigne une position angulaire appropriée à ces attentes. Puis, le chirurgien bloque la pièce 30 par rapport à la pièce 20, pour figer le basculement relatif entre ces pièces. De cette façon, la suite de l'intervention chirurgicale se déroulera alors que la rotation externe du fémur F sera ajustée fixement sur la valeur de rotation prédéterminée préopératoire.

Dans le mode de réalisation considéré ici, la mise en oeuvre de la variante d'utilisation évoquée juste ci-dessus repose sur la possibilité de visser le téton 35 autour du pion 34, de manière à enserrer jusqu'à bloquer, de manière réversible, la pièce d'appui fémoral 20 contre l'extrémité supérieure du corps 31 de la pièce 30.

Un troisième critère en fonction duquel la rotation externe du fémur F peut être ajustée est montré sur la figure 3B. Ce troisième critère se rapporte à ce qui est couramment appelé la ligne de Whiteside, qui, en anatomie, correspond à la ligne de fond antéropostérieure de l'échancrure intercondylienne du fémur. Ainsi, pour ajuter la rotation externe du fémur, le chirurgien compare visuellement la position de la ligne de Whiteside et une pointe 37 dont est munie, ici rigidement, la pièce interposée 30. Cette pointe 37 s'étend le long du corps 31, suivant une direction parallèle à l'axe X-X, en étant situé en travers d'une ouverture traversante qui est délimitée par le corps 31 pour permettre une observation antéropostérieure à travers ce corps. De même, pour permettre au chirurgien d'observer la pointe 37, le pilier 13 est ajouré de part en part suivant une direction antéropostérieure, comme bien visible sur la figure 3B.

La figure 3C illustre un quatrième critère en fonction duquel le chirurgien peut ajuster la rotation externe du fémur F. La mise en oeuvre de ce critère repose sur la ligne biépycondylienne, c'est-à-dire la ligne qui, en anatomie, relie les points extrêmes médial et latéral des condyles du fémur. En pratique, le chirurgien va comparer visuellement la ligne biépycondilienne et une tige 38 qui est rapportée fixement la pièce interposée 30. Cette tige 38 s'étend en longueur suivant une direction perpendiculaire, à la fois, à l'axe X-X et à l'axe Y-Y. Dans l'exemple de réalisation considéré ici, la tige 38 est en fait constituée de deux parties de tige distinctes 38₁ et 38₂, qui sont respectivement rapportées sur les côtés médial et latéral du corps 31 : une extrémité longitudinale de chaque partie de tige 38₁, 38₂ est reçue et solidarisée, par exemple par vissage, dans un trou borgne complémentaire 39_{1,} 39₂ (figure 1E) délimité par le corps 31. Ainsi, en service, les parties de tige 38₁ et 38₂ matérialisent la tige 38 qui s'étend en correspondance visuelle avec le côté distal du fémur F, permettant au chirurgien d'apprécier si cette tige 38 est alignée ou non avec la ligne biépycondilienne.

Enfin, sur la figure 3D, l'instrumentation chirurgicale 1 comprend, en plus des pièces 10, 20 et 30, une cale 50 interposée fixement entre la palette 21 de la pièce d'appui fémoral 20 et le condyle postérieur externe du fémur F. On comprend que, eu égard à l'épaisseur de la cale 50, c'est-à-dire à sa dimension suivant la direction de l'axe X-X, la présence de cette cale modifie la position de rotation externe du fémur. En fait, l'ajustement de rotation externe du fémur est réalisé par rapport au condyle interne dont la position, par rapport au tibia, est fixe, en étant supporté par la palette 21.

En pratique, le chirurgien a à sa disposition un jeu de plusieurs cales similaires à la cale 50, mais qui présente des épaisseurs respectives différentes selon la direction de l'axe X-X : selon la cale choisie parmi le jeu, qui est interposée entre le condyle postérieur externe et la palette 21, la rotation externe du fémur est ainsi ajustée en fonction d'un critère lié à la position du condyle interne.

Ainsi, à l'issue du troisième temps opératoire, le chirurgien a ajusté la rotation externe du fémur F en fonction d'un critère parmi le groupe incluant les cinq critères précités, à savoir la mesure peropératoire de la valeur de cette rotation externe, la fixation préopératoire d'une valeur de rotation prédéterminée, la position de la ligne de Whiteside, la position de la ligne biépycondilienne et la position du condyle interne. La mise en oeuvre de l'un quelconque de ces cinq critères, voire de plusieurs d'entre eux de manière successive, est réalisée à l'aide de la seule et même instrumentation 1, en particulier à l'aide des pièces 10, 20 et 30, ainsi que, le cas échéant, du jeu des cales 50. Dans tous les cas, cette mise en oeuvre est réalisée alors que le tibia T et le fémur F sont écartés l'un de l'autre, sous l'action de la pince de distraction 40 dont les extrémités distales 43 et 44 sont respectivement liées, en mouvement, au moins suivant l'axe X-X, aux pièces d'appui tibial 10 et d'appui fémoral 20.

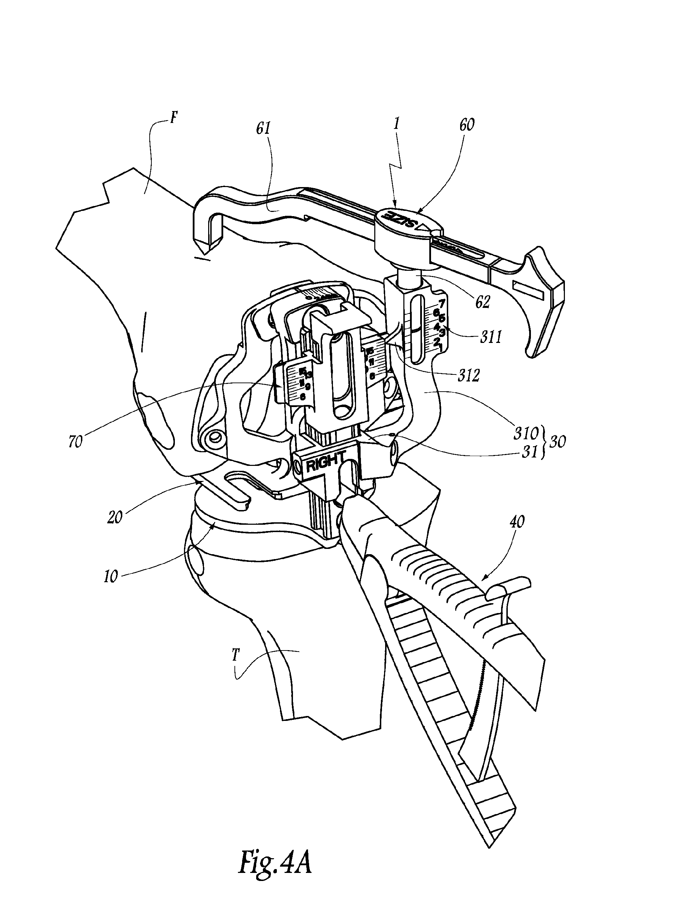

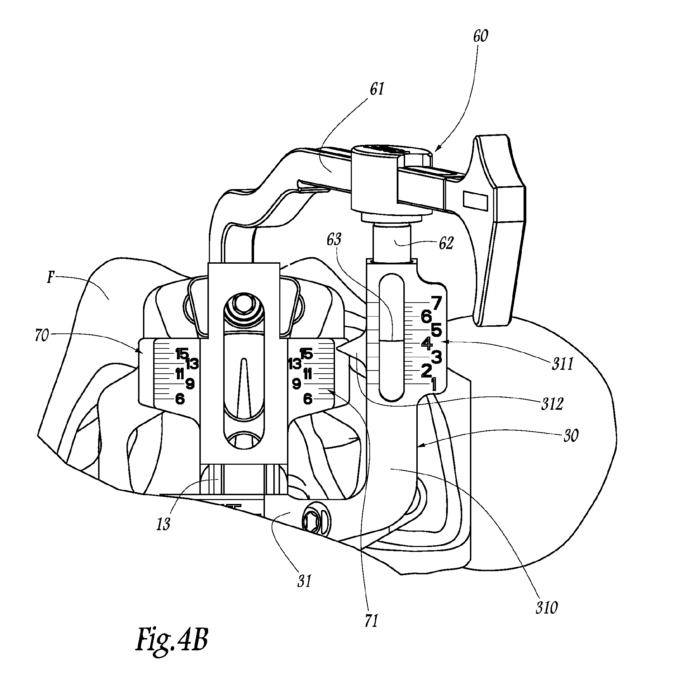

Dans un quatrième temps opératoire, le chirurgien va mesurer la dimension antéropostérieure de l'extrémité inférieure du fémur, en vue de choisir un composant fémoral de la prothèse de genou à poser parmi plusieurs composants fémoraux dont les dimensions antéropostérieures respectives sont différentes. Pour ce faire, le chirurgien utilise une pièce 60 de palpation de la corticale antérieure du fémur. Comme montré sur les figures 4A et 4B, cette pièce 60 est conçue pour être rapportée sur la pièce interposée 30. Plus précisément, la pièce de palpation 60 comprend un bras allongé 61 dont l'une des extrémités est conformée pour venir s'appuyer contre la corticale antérieure du fémur tandis que, dans sa partie courante, le bras 61 est muni d'un doigt transversal 62 adapté pour être reçu de manière complémentaire dans une cheminée 310 solidaire du corps 31. Cette cheminée 310 s'étend parallèlement à l'axe X-X et présente, tournée vers le chirurgien, des graduations 311, allant ici de « 1 » à « 7 », respectivement associées à autant de tailles d'un composant fémoral prothétique. Ainsi, comme montré sur la figure 4B, le chirurgien compare visuellement une référence 63, ici sous forme d'un trait, porté fixement par le doigt 62 de la pièce de palpation 60 et, les graduations 311, pour déterminer la taille du composant fémoral prothétique à choisir.

Dans l'exemple représenté sur la figure 4B, la taille du composant fémoral prothétique est évaluée entre les graduations « 4 » et « 5 ». Par conséquent, si le chirurgien choisit une taille de composant fémoral « 4 », le genou du patient présentera, après implantation des composants fémoral et tibial de la prothèse de genou, une certaine laxité puisque, en flexion, l'encombrement prothétique sera légèrement plus petit que l'espace disponible entre le tibia et le fémur, sauf à ce que le chirurgien implante un composant tibial prothétique un peu plus épais. A l'inverse, si le chirurgien choisit une taille de composant fémoral « 5 », le genou du patient sera, après implantation de la prothèse, plus serré, puisque l'encombrement prothétique sera légèrement supérieur à l'espace inter-osseux disponible, sauf à ce que le chirurgien réalise un relâchement ligamentaire.

Avantageusement, les graduations 311 permettent de quantifier l'écart entre la dimension antéropostérieure du composant fémoral prothétique choisi et la dimension antéropostérieure du fémur non encore prothésé, grâce à des sous-graduations, notamment millimétriques, entre les graduations associées aux tailles d'implant « 1 » à « 7 ». Ainsi, en reprenant l'exemple montré sur la figure 4B, l'écart avec la taille « 4 » est de + 1 mm, tandis que l'écart avec la taille « 5 » est de - 2 mm.

Cette quantification de l'écart peut alors être corrélée avec une mesure de l'espace inter-osseux disponible entre le tibia T et le fémur F lorsque ces os sont distraits ou mis en tension l'un vis-à-vis de l'autre. Pour ce faire, l'instrumentation 1 comprend avantageusement une pièce de mesure 70 adaptée pour être rapportée sur la pièce d'appui tibial 10, ici de manière coulissante sur le pilier 13. Cette pièce de mesure 70 est dimensionnée pour, après avoir été rapportée sur la pièce 10, coopérer par correspondance visuelle avec une référence 312 de la pièce interposée 30, située ici le long de la cheminée 310. Cette référence 312 se trouve positionnée en regard de graduations 71 dont est munie la pièce de mesure 70, pour lire directement une valeur parmi ces graduations, correspondant à l'écartement relatif, suivant l'axe X-X, du tibia T et du fémur F sous l'action des pièces d'appui tibial 10 et d'appui fémoral 20 entraînées par la pince de distraction 40. Ainsi, dans l'exemple montré sur la figure 4B, l'écartement relatif du tibia et du fémur est mesuré à 16 mm. Cette indication permet au chirurgien de choisir l'épaisseur du composant tibial prothétique qu'il va implanter ultérieurement.

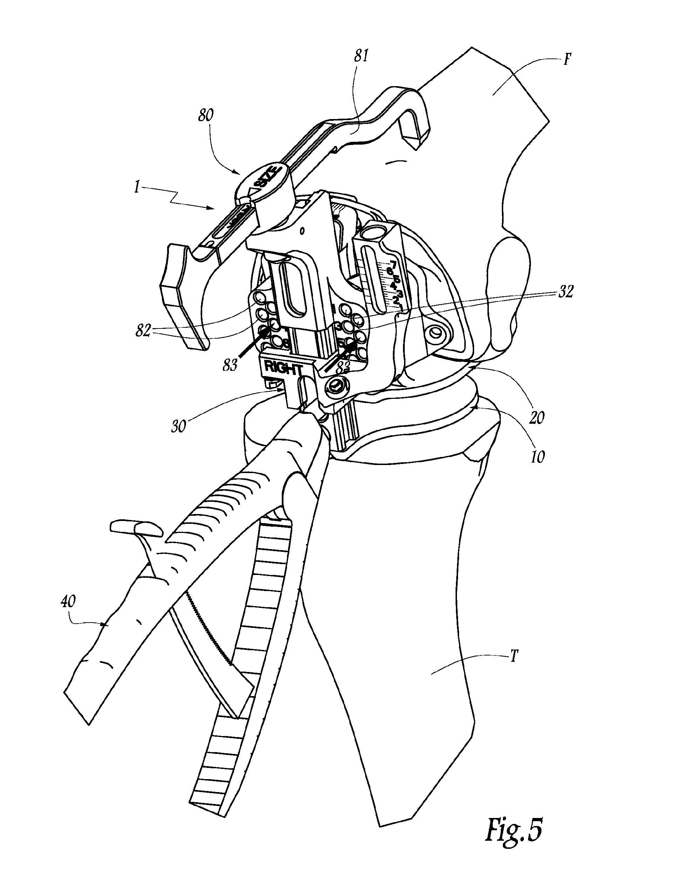

Dans un cinquième temps opératoire, illustré à la figure 5, le chirurgien va préparer le positionnement d'un bloc de coupe fémorale qui sera mis en oeuvre lors du sixième temps opératoire décrit plus loin. Pour ce faire, le chirurgien utilise un guide de positionnement 80 adapté pour être rapporté sur la pièce d'appui tibial 10, avantageusement en étant rapporté de manière coulissante autour du pilier 13. Dans le mode de réalisation considéré sur la figure 5, le guide de positionnement 80 inclut un bras 81 de palpation de la corticale antérieure du fémur F, ce qui implique que le positionnement du bloc de coupe fémorale évoqué ci-dessus est réalisé par rapport à la corticale antérieure du fémur. Autrement dit, le bloc de coupe est positionné, par le guide 80, en prenant comme référence la corticale antérieure du fémur. De plus, le guide de positionnement 80 est muni, respectivement sur ses côtés distal et latéral, de deux séries de sept trous traversants 82, respectivement repérés par des références numériques « 1 » à « 7 ». De cette façon, le chirurgien repère, dans chaque série de trous, le trou associé à la référence numérique correspondant à la taille du composant fémoral prothétique qu'il a choisie à l'issue du quatrième temps opératoire. A titre d'exemple, dans l'hypothèse où, à l'issue du quatrième temps opératoire, le chirurgien a sélectionné la taille « 4 », il utilise la paire de trous 82 associés à la référence « 4 » pour y introduire deux broches qui s'ancrent dans le côté distal des condyles des fémurs, comme indiqué par les flèches 83 sur la figure 5. En pratique, les trous 82 peuvent être utilisés pour, préalablement à la mise en place des broches précitées, guider un foret de perçage, afin de réaliser dans le fémur une cavité de réception de ces broches.

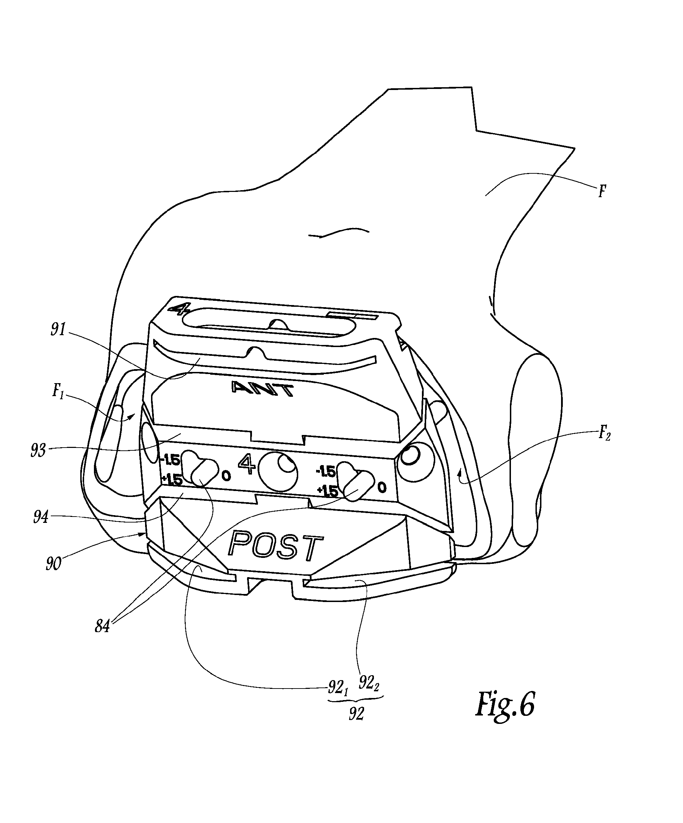

Enfin, lors d'un sixième temps opératoire illustré à la figure 6, le chirurgien plaque un bloc de coupe fémorale 90 contre le côté distal de l'extrémité inférieure du fémur F. Le positionnement antéropostérieur de ce bloc de coupe est réalisé à l'aide des broches, référencées 84 sur la figure 6, mises en place à l'issue du cinquième temps opératoire. De manière connue en soi, le bloc de coupe 90 inclut plusieurs fentes de coupe, dans chacune desquelles une lame de scie est introduite pour réséquer l'extrémité inférieure du fémur, dans un plan en affleurement de cette fente. En particulier, le bloc de coupe 90 comprend une fente de coupe antérieure 91 et une fente de coupe postérieure 92, qui, ici, est constituée d'une sous-fente médiale 92₁ et d'une sous-fente latérale 92₂. Avantageusement, le bloc de coupe 90 inclut ici également deux autres fentes, référencées 93 et 94 sur la figure 6, qui permettent de réaliser des chanfreins entre les surfaces distales F₁ et F₂ du fémur et, d'une part, la surface de résection obtenue à l'aide de la fente antérieure 91 et les surfaces de résection obtenues à l'aide des sous-fentes postérieures 92₁ et 92₂.

On comprend que le bloc de coupe 90 est dimensionné pour que, en étant positionné sur le fémur F à l'aide des broches 84, sa fente antérieure 91 et sa fente postérieure 92 permettent de réaliser des surfaces de résection du fémur, positionnées vis-à-vis de ce dernier, avec pour référence la corticale antérieure du fémur. Bien entendu, la distance antéropostérieure séparant les fentes 91 et 92 correspondent à la taille antéropostérieure du composant fémoral prothétique à implanter de sorte que, en pratique, le chirurgien dispose d'un jeu de plusieurs blocs de coupe, respectivement associés à chacune des tailles de composant «1 »à«7»,

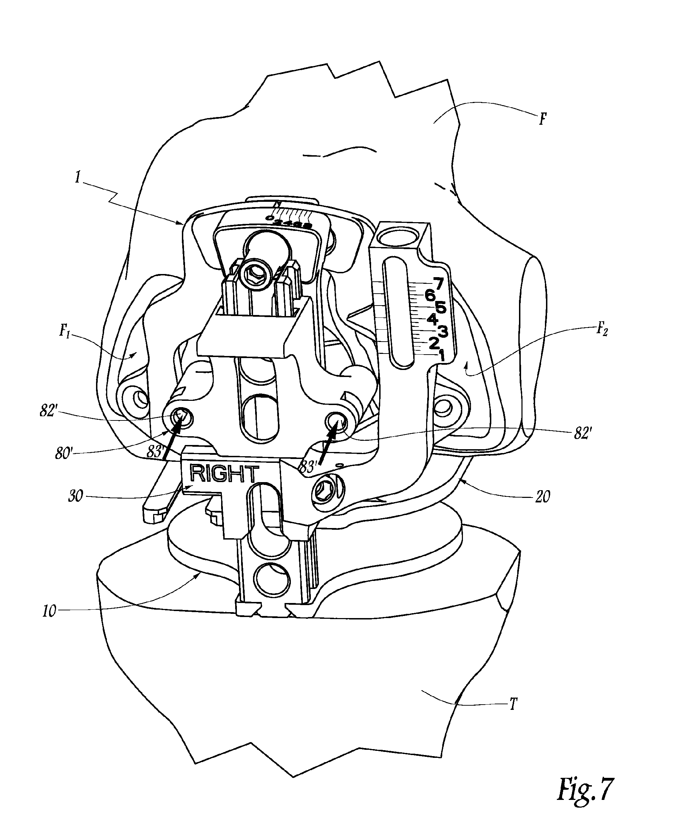

Avantageusement, plutôt que de positionner les blocs de coupe 90 en prenant comme référence de positionnement la corticale antérieure du fémur, ce qui correspond à une première option de mise en oeuvre, une seconde option de l'instrumentation 1 correspond à positionner ces blocs de coupe en prenant comme référence les condyles postérieurs du fémur. Pour ce faire, le chirurgien n'utilise pas, lors du cinquième temps opératoire, le guide de positionnement 80, mais un guide de positionnement 80', comme montré sur la figure 7. Ce guide de positionnement 80' est conçu pour être rapporté sur la pièce d'appui tibial 10, ici de manière coulissante autour du pilier 13, jusqu'à prendre appui, suivant l'axe X-X, sur la pièce d'appui fémoral 20, avec interposition de la pièce interposée 30. De cette façon, le guide de positionnement 80' est placé, vis-à-vis du fémur F, en étant repérable par rapport aux condyles postérieurs du fémur, en appui contre la palette 21 de la pièce 20. Par conséquent, moyennement un dimensionnement adéquat de ce guide 80', ce dernier permet de mettre en place dans les surfaces distales F₁ et F₂ du fémur F deux broches similaires aux broches 84, comme indiqué par les flèches 83' sur la figure 7. Ces broches sont ensuite utilisées, au cours du sixième temps opératoire, pour mettre en place l'un des blocs de coupe 90, à savoir celui correspond à la taille de composant fémoral prothétique retenue. On remarquera que, ici, à la différence du guide de positionnement 80, le guide de positionnement 80' n'inclut qu'une paire de trous 82' pour mettre en place les broches précitées : cela s'explique par le fait qu'on a prévu ici que la dimension antéropostérieure des différents blocs de coupe 90, entre leur fente postérieure 92 et leurs trous de réception des broches, est constante quelle que soit la taille de ce bloc de coupe (tandis que la dimension antéropostérieure entre leurs trous de réception des broches et leur fente antérieure 91 varie selon la taille des blocs de coupe).

Ainsi, grâce aux guides de positionnement 80 et 80', l'instrumentation 1 permet, en per-opératoire, de choisir la référence de positionnement, soit antérieure, soit postérieure, pour le bloc de coupe 90 utilisée lors du sixième temps opératoire.

De manière plus générale, au vu de ce qui précède, on comprend que l'instrumentation chirurgicale 1 permet de combiner plusieurs techniques opératoires avec un seul et même ensemble de composants pré-assemblés que sont la pièce d'appui tibial 10, la pièce d'appui fémoral 20, la pièce interposée 30 et la pince de distraction 40. En outre, pour chacune de ces techniques opératoires, l'instrumentation 1 permet de régler à volonté la rotation externe du fémur F par rapport au tibia T. Par ailleurs, l'instrumentation 1 permet de réaliser la préparation à la pose de la prothèse de genou en étant rapportée de manière solide et stable sur le fémur, sans qu'il soit nécessaire de démonter puis de remonter une partie de l'instrumentation. Par ailleurs, on remarquera que les différents composants de l'instrumentation 1 ont été conformés pour donner au chirurgien une bonne visibilité de l'extrémité inférieure du fémur F et de l'extrémité supérieure du tibia T.

Divers aménagements et variantes à l'instrumentation 1 décrite jusqu'ici sont par ailleurs envisageables. A titre d'exemples :
- à titre optionnel, la pièce interposée 30 peut être verrouillée dans une position coulissée choisie, par rapport à la pièce d'appui tibial 10 ; pour ce faire, on peut par exemple utiliser une vie de blocage référencée 313 sur la figure 1E, qui est rapportée à travers le corps 31, jusqu'à coincer le pilier 13 dans le trou 32 ; de cette façon, l'espace en flexion, entre le tibia T et le fémur F, est arbitrairement fixé, en étant avantageusement quantifié de manière précise grâce à la pièce de mesure 70 ; et/ou
- dans la description qui précède, l'instrumentation 1 a été mise en oeuvre sur un genou droit ; un genou gauche peut être traité par une instrumentation similaire, étant remarqué que, avantageusement, cet instrumentation similaire inclut les mêmes composants que l'instrumentation 1, excepté la pièce interposée 30 remplacée par une pièce interposée symétrique à la pièce 30 décrite jusqu'ici, par rapport à un plan sagittal au patient.

## Revendications

1. Instrumentation chirurgicale (1) pour la préparation à la pose d'une prothèse de genou, comportant une pièce d'appui tibial (10) et une pièce d'appui fémoral (20), adaptées pour s'appuyer respectivement contre l'extrémité supérieure du tibia (T) et contre les condyles postérieurs du fémur (F), de manière à écarter l'un de l'autre le tibia et le fémur lorsque le genou est fléchi, **caractérisée en ce qu'**elle comporte en outre des moyens (30, 50) de montage et de couplage de la pièce d'appui fémoral sur la pièce d'appui tibial, qui sont conçus pour, alors que les pièces d'appui tibial et d'appui fémoral sont appuyées contre le tibia et le fémur, ajuster la rotation externe du fémur sélectivement en fonction d'un critère relatif à une technique de pose parmi au moins deux critères appartenant à un groupe qui inclut la mesure peropératoire de la valeur de la rotation externe, la fixation préopératoire à une valeur de rotation prédéterminée, la position de la ligne de Whiteside, la position de la ligne biépycondylienne et la position du condyle interne.

2. Instrumentation suivant la revendication 1, **caractérisée en ce que** les moyens de montage et de couplage (30, 50) sont conçus pour ajuster la rotation du fémur en fonction d'un critère sélectionné parmi au moins trois des critères dudit groupe.

3. Instrumentation suivant la revendication 1, **caractérisée en ce que** les moyens de montage et de couplage (30, 50) sont conçus pour ajuster la rotation du fémur (F) en fonction d'un critère sélectionné parmi au moins quatre des critères dudit groupe.

4. Instrumentation suivant la revendication 1, **caractérisée en ce que** les moyens de montage et de couplage (30, 50) sont conçus pour ajuster la rotation du fémur (F) en fonction d'un critère sélectionné parmi les cinq critères dudit groupe.

5. Instrumentation suivant l'une quelconque des revendications précédentes, **caractérisée en ce que** les moyens de montage et de couplage comprennent une pièce (30) interposée mécaniquement entre la pièce d'appui tibial (10) et la pièce d'appui fémoral (20), laquelle pièce interposée est montée sur la pièce d'appui tibial de manière coulissante suivant un axe de coulissement (X-X) qui s'étend selon la direction d'écartement relatif du tibia et du fémur sous l'action des pièces d'appui tibial et d'appui fémoral, tandis que la pièce d'appui fémoral est montée sur cette pièce interposée de manière basculante autour d'un axe de basculement (Y-Y) qui est sensiblement perpendiculaire à l'axe de coulissement et qui, lorsque les pièces d'appui tibial et d'appui fémoral sont appuyées contre le tibia et le fémur alors que le genou est fléchi, s'étend selon la direction longitudinale du fémur.

6. Instrumentation suivant la revendication 5, **caractérisée en ce que** la pièce interposée (30) est munie, éventuellement de façon amovible, de graduations (36) qui sont réparties autour de l'axe de basculement (Y-Y) et qui sont en correspondance visuelle avec une référence (26) dont est munie la pièce d'appui fémoral (20), de manière à mesurer de façon peropératoire la valeur de la rotation externe du fémur (F).

7. Instrumentation suivant l'une des revendications 5 ou 6, **caractérisée en ce que** les moyens de montage et de couplage comprennent un mécanisme de blocage réversible (34, 35) adapté pour immobiliser par rapport à la pièce interposée (30) la pièce d'appui fémoral (20) quelle que soit la position basculée de cette dernière autour de l'axe de basculement (Y-Y), de manière à fixer la rotation externe du fémur (F) à une valeur de rotation prédéterminée préopératoire.

8. Instrumentation suivant l'une quelconque des revendications 5 à 7, **caractérisée en ce que** la pièce interposée (30) est munie, éventuellement de façon amovible, d'une pointe (37) qui s'étend en longueur suivant une direction parallèle à l'axe de coulissement (X-X) et qui, lorsque les pièces d'appui tibial (10) et d'appui fémoral (20) sont appuyées sur le tibia (T) et le fémur (F) alors que le genou est fléchi, est située en correspondance visuelle avec le coté distal du fémur de manière à ajuster la valeur de la rotation externe du fémur en fonction de la position de la ligne de Whiteside.

9. Instrumentation suivant l'une quelconque des revendications 5 à 8, **caractérisée en ce que** la pièce interposée (30) est munie, éventuellement de façon amovible, d'une tige (38) qui s'étend en longueur suivant une direction perpendiculaire à la fois à l'axe de coulissement (X-X) et à l'axe de basculement (Y-Y) et qui, lorsque les pièces d'appui tibial (10) et d'appui fémoral (20) sont appuyées sur le tibia (T) et le fémur (F) alors que le genou est fléchi, est située en correspondance visuelle avec le coté vistal du fémur de manière à ajuster la rotation externe du fémur en fonction de la position de sa ligne biépycondylienne.

10. Instrumentation suivant l'une quelconque des revendications 5 à 9, **caractérisée en ce que** les moyens de montage et de couplage comprennent un jeu de cales (50), qui sont chacune adaptées pour être placées fixement en interposition entre la pièce d'appui fémoral (20) et le condyle externe du fémur (F) et qui présentent des épaisseurs respectives différentes selon la direction de cette interposition, de manière à ajuster la rotation externe du fémur en fonction de la position du condyle interne.

11. Instrumentation suivant l'une quelconque des revendications précédentes, **caractérisée en ce qu'**elle porte en outre une pièce (60) de palpation de la corticale antérieure du fémur (F), cette pièce de palpation étant adaptée pour coopérer avec les moyens de montage et de couplage (30, 50), alors que ces derniers sont en service, de manière, à la fois, à choisir un composant fémoral de la prothèse de genou à poser parmi plusieurs composants fémoraux dont les dimensions antéropostérieures respectives sont différentes, et à mesurer l'écart, éventuellement nul, entre la dimension antéropostérieure du composant fémoral choisi et une dimension antéropostérieure correspondante du fémur non encore prothésé.

12. Instrumentation suivant l'une quelconque des revendications précédentes, **caractérisée en ce qu'**elle comporte en outre des moyens (70) de mesure de l'écartement relatif entre le tibia (T) et le fémur (F) sous l'action des pièces d'appui tibial (10) et d'appui fémoral (20).

13. Instrumentation suivant l'une quelconque des revendications précédentes, **caractérisée en ce que** la pièce d'appui tibial (10) est conçue pour supporter sélectivement un premier guide (80) de positionnement d'un bloc de coupe fémorale (90), dont la position est repérée vis-à-vis du fémur (F) par une référence antérieure de ce dernier, notamment par palpation de la corticale antérieure du fémur, et un second guide (80') de positionnement d'un bloc de coupe fémorale, dont la position est repérée vis-à-vis du fémur par une référence postérieure de ce dernier, notamment par appui sur la pièce d'appui fémoral (20), avec interposition des moyens de montage et de couplage (30, 50).

14. Instrumentation suivant la revendication 13, **caractérisée en ce qu'**elle comporte lesdits premier (80) et second (80') guides de positionnement.

15. Instrumentation suivant l'une des revendications 13 ou 14, **caractérisée en ce qu'**elle comporte au moins un bloc de coupe fémorale (90) conçu pour être positionné indifféremment par le premier guide (80) et par le second guide (80').
